# EUROPÄISCHE PATENTSCHRIFT

(11) **EP 2 111 379 B1**
(45) Veröffentlichungstag und Bekanntmachung des Hinweises auf die Patenterteilung: **25.03.2015**
(21) Anmeldenummer: 08715433.2
(22) Anmeldetag: 06.01.2008
(51) Int. Cl.: C02F 3/34, C02F 1/40, C02F 101/32, C02F 103/08

(54) **BIOREMEDIATIONSVERFAHREN ZUM BESCHLEUNIGTEN BIOLOGISCHEN ABBAU VON PETROLEUM-KOHLENWASSERSTOFFEN IN DEN POLAREN MEEREISBEDECKTEN REGIONEN UND BAKTERIEN- UND ENZYMGEMISCHE ALS MITTEL ZUR VERFAHRENSDURCHFÜHRUNG**
BIOREMEDIATION METHOD FOR ACCELERATED BIOLOGICAL DEGRADATION OF PETROLEUM HYDROCARBONS IN SEA ICE-COVERED POLAR REGIONS, AND BACTERIA AND ENZYME MIXTURES AS AGENTS FOR CARRYING OUT SAID METHOD
PROCÉDÉ DE BIOREMÉDIATION PERMETTANT UNE BIODÉGRADATION ACCÉLÉRÉE D'HYDROCARBURES D'HUILE MINÉRALE DANS LES RÉGIONS POLAIRES COUVERTES PAR UNE BANQUISE, ET MÉLANGES DE BACTÉRIES ET D'ENZYMES COMME MOYENS POUR RÉALISER CE PROCÉDÉ

(30) Priorität: 21.01.2007 DE 102007003644
(43) Veröffentlichungstag der Anmeldung: 28.10.2009
(73) Patentinhaber: Alfred-Wegener-Institut Helmholtz-Zentrum für Polar- und Meeresforschung, 27570 Bremerhaven (DE)
(72) Erfinder: HELMKE, Elisabeth, 27572 Bremerhaven (DE); GERDES, Birte, 95444 Bayreuth (DE); JÜRGENS, Jutta, 27570 Bremerhaven (DE); REUTER, Kristine, 27607 Langen (DE)
(86) Internationale Anmeldenummer: PCT/DE2008/000018
(87) Internationale Veröffentlichungsnummer: WO 2008/089718

(56) Entgegenhaltungen:
- DE-A1- 19 652 580
- RU-C1- 2 107 722
- UTA DEPPE ET AL: "Degradation of crude oil by an arctic microbial consortium" EXTREMOPHILES ; LIFE UNDER EXTREME CONDITIONS, SPRINGER-VERLAG, TO, Bd. 9, Nr. 6, 1. Dezember 2005 (2005-12-01), Seiten 461-470, XP019374120 ISSN: 1433-4909
- RIKE A G ET AL: "In situ biodegradation of hydrocarbons in arctic soil at sub-zero temperatures-field monitoring and theoretical simulation of the microbial activation temperature at a Spitsbergen contaminated site" COLD REGIONS SCIENCE AND TECHNOLOGY, ELSEVIER, Bd. 41, Nr. 3, 1. März 2005 (2005-03-01), Seiten 189-209, XP004736480 ISSN: 0165-232X
- MARGESIN R ET AL: "REVIEW BIOLOGICAL DECONTAMINATION OF OIL SPILLS IN COLD ENVIRONMENTS" JOURNAL OF CHEMICAL TECHNOLOGY AND BIOTECHNOLOGY, BLACKWELL SCIENTIFIC PUBLICATIONS. OXFORD, GB, Bd. 74, Nr. 5, 1. Mai 1999 (1999-05-01), Seiten 381-389, XP000877893 ISSN: 0268-2575
- J. C. PRISCU, C. H. FRITSEN, E. A. ADAMS, S. J. GIOVANNONI, H. W. PAERL, C. P. MCKAY, P. T. DORAN, D. A. GORDON, LANOIL, PINCKNEY: "Perennial Antarctic Lake Ice: An Oasis for Life in a Polar Desert" SCIENCE, Bd. 280, 26. Juni 1998 (1998-06-26), Seiten 2095-2098, XP002492565

## Beschreibung

Die Erfindung bezieht sich auf ein Bioremediationsverfahren zum beschleunigten biologischen Abbau von Petroleum-Kohlenwasserstoffen in den polaren meereisbedeckten Regionen durch Bioaugmentation mit exogenen kohlenwasserstoffabbauenden Bakterien und durch Zugabe von Nährstoffen sowie auf Bakterien- und Enzymgemische als Mittel zur Durchführung des Bioremediationsverfahrens.

Die zunehmende Erschöpfung der momentan genutzten Ölvorkommen (Petroleum-Kohlenwasserstoffe) führt zurzeit zu einer Hinwendung zu Extremstandorten bei der Ölprospektion. Dabei treten zunehmend auch der Arktische Ozean mit seinen Randmeeren, die arktischen Schelfbereiche und die arktischen Permafrost-Gebiete in den Fokus des Interesses, wo ca. 25% der auf der Erde befindlichen Öl- und Gas-Vorkommen vermutet werden. An einigen Stellen wird bereits in nicht unerheblichem Maße Öl gefördert.

Der zentrale arktische Ozean ist ganzjährig mit zum Teil dickem Meereis bedeckt und die arktischen Randmeere bzw. Schelf- und Küstenregionen weisen Treibeis oder eine geschlossene Meereisbedeckung zumindest während der kälteren Jahreszeit auf. Förderplattformen, die unmittelbar in diesen Gebieten eingerichtet werden, unterliegen besonderen Beanspruchungen durch die Eisdrift. Weiteres Problem ist die Materialermüdung durch sehr niedrige Temperaturen. Dieses gilt z.B. für die untermeerischen Pipelines, die bis zu den Liegeplätzen der Schiffe auf dem Schelfgebiet führen. Auch der Abtransport des Öls per Schiff durch das Eis birgt erhöhte Gefahren, sodass das Risiko von Ölkontaminationen oder Schiffshavarien in diesem Gebiet deutlich größer als in gemäßigten oder tropischen Breiten ist. Neben den singulären Ölverschmutzungen, insbesondere durch Unfälle und Havarien, besteht in den polaren Eisregionen also auch eine große Gefahr von kontinuierlichen Ölverschmutzungen, beispielsweise durch Produktion von Öl an Förderplattformen oder durch den Transport von Öl beim Beladen oder durch Leckagen in den bruchgefährdeten Pipelines.

Die bisher im freien Wasser eingesetzten Ölbeseitigungsmaßnahmen sind in den polaren Eisregionen nur begrenzt einsetzbar, da sich die Verteilung von Öl im Eis von jener im Wasser signifikant unterscheidet. Nur Eisbrei verhält sich ähnlich wie Wasser. Problematisch bei der Entwicklung von Verfahren zur Ölbeseitigung in meereisbedeckten Regionen sind die unterschiedlichen Eisverhältnisse. Eine einheitliche Eisbedeckung und damit ein Verfahren, dass generell im Eis eingesetzt werden kann, gibt es nicht. Eisverhältnisse variieren von Plättchen-Eis, Brei-Eis, über junges Pfannkuchen-Eis, größere Schollen bis hin zu einer geschlossenen Eisdecke. Die Maßnahmen der Eisbeseitigung sind nicht nur von der Eiskonsistenz, Schollengröße und Schollendicke, sondern auch von der Dynamik des Eisfeldes, vom Wetter und vom Wellengang des Wassers zwischen den Schollen abhängig. Der Ölteppich kann unter das Eis gelangen, über die Eisschollen verschmieren, in das Eis eingefroren werden oder sich mit dem Brei-Eis homogen vermischen. Generell gilt, sobald das Öl in das Eis gelangt, wird eine Auftrennung zwischen Öl und Eis sehr schwierig. Einfacher ist es noch, wenn sich das Öl zwischen Eisschollen im Wasser konzentriert, obgleich auch dann nicht alle im Wasser üblichen Verfahren eingesetzt werden können, da die meisten Gerätschaften im Bereich von Eisschollen nicht manövrierfähig sind (z.B. Ölbarrieren).

### STAND DER TECHNIK

Bisher sind noch keine kleineren oder größeren Unfälle in den vom Meereis bedeckten Gebieten bekannt geworden. Da das Risiko einer Ölkontamination aber zunimmt und die Selbstreinigungskraft dieser Ökosysteme wegen der dort herrschenden sehr niedrigen Temperaturen bekanntermaßen deutlich geringer ist als an anderen Standorten, wird die Entwicklung von effektiven Maßnahmen und Verfahren zur Reinigung von Ölverschmutzungen in meereisbedeckten Gebieten immer dringlicher. In einem Bericht des EU-Projektes ARCOP, Veröffentlichung I aus Juli 2004 (ARCOP D4.2.1.1 (a), Kapitel 4 "Oil spill response -present alternatives for ice covered water" pp 29-88), wird der derzeitige Stand der Technik bei der Ölbergung in eisbedeckten Gebieten umfassend dargestellt. Mögliche Verfahren (siehe unten) sowie deren Nachteile und Vorteile werden vorgestellt. Einige der vorgestellten Verfahren und Gerätschaften wurden bisher in kleinem Rahmen getestet (Studien), zu ernsthaften Einsätzen kam es aber noch nicht.
- Mechanische Methoden, die alle auf eine Art "Waschprinzip" hinauslaufen, machen den größten Teil der angedachten Ölbeseitigungsverfahren aus. Diese Verfahren eignen sich jedoch nur für die Säuberung kleinerer Eis-Schollen. Reste von Öl bleiben im Eis zurück. Die Biologie der Umwelt wird bei allen mechanischen Prinzipien stark in Mitleidenschaft gezogen, meist sogar ganz zerstört. Die Regeneration verläuft äußerst langsam.
- Das Verbrennen von Öl im Eis, das so genannte "in situ burning" ist ein Verfahren, welches nur bei geringer und dann wieder bei sehr hoher Eisbedeckung eingesetzt werden kann. Der Ölanteil im Eis muss 25% übersteigen, damit das Öl abbrennt. Die Ölbeseitigung ist schnell und effektiv. Nachteilig ist bei diesem Verfahren die entstehende Hitze, die das Ökosystem komplett vernichtet, sodass danach kaum Aussicht besteht, dass Restverschmutzungen natürlich biologisch abgebaut werden. Darüber hinaus können die Emissionen und Verbrennungsreste auch in weiterer Entfernung vom Verbrennungsort toxisch wirken.
- Chemische Verfahren sind im Eis nur bedingt einsetzbar, da eine effektive Vermischung fehlt. Diese ist nur dann gegeben, wenn sich wenige Eisschollen in turbulentem Wasser befinden. Viele der chemischen Dispersionsmittel sind außerdem toxisch, daher in Deutschland zum größten Teil verboten. Außerdem wird das Öl nicht wirklich beseitigt, sondern das Problem nur verlagert, da sich dispergiertes Öl besser verteilt.
- Biologische Verfahren sind im Eis theoretisch einsetzbar, praktisch wurden sie bisher jedoch nicht berücksichtigt, da vom Fachmann aufgrund der niedrigen unphysiologischen Temperaturen im Eis ein effektiver biologischer Ölabbau ausgeschlossen wird. Zwar konnte ein geringer Besatz an Kohlenwasserstoffabbauern im Eis festgestellt werden, jedoch handelte es sich dabei lediglich um kurzkettige Alkanabbauer (vergleiche **Veröffentlichung II** von B. Gerdes et al. "Influence of crude oil on changes of bacterial communities in Artic sea-ice", FEMS Microbiology Ecology 53 (2005) pp129-139). Die Beimpfung mit Ölabbauern im Eis stellt sich ebenfalls problematisch dar, da die Temperatur- und Salzbedingungen nicht nur extrem, sondern auch stark variierend sind.

Allgemein sind biologische Verfahren, auch Bioremediationsverfahren genannt, sehr umweltschonende Verfahren, die im Stand der Technik an wärmeren Standorten, insbesondere bei der Sanierung von Böden, häufig eingesetzt werden. Dabei wird die natürlich vorkommende, ölabbauende Mikrobengemeinschaft durch die Ausbringung von Nährstoffen, die der Beseitigung des Stickstoff- und Phosphatmangels dient, in ihrer Entwicklung gefördert und der natürliche Abbauprozess beschleunigt. Diese Technik wurde bisher nicht nur erfolgreich in Böden, sondern auch in küstennahem Wasser, sogar in kaltem Wasser, z.B. bei der Exxon-Valdez-Havarie in den arktischen Gewässern vor der Küste Alaskas, eingesetzt. Neben der Zuführung von Nährstoffen wurde auch mit der Zugabe von ölabbauenden Bakterien experimentiert. Dieses Beimpfungsverfahren hat sich allerdings bisher nur in geschlossenen Anlagen oder Behältern (ex situ Verfahren, z.B. zur Sanierung von Bodenabtrag) und nicht in der freien Natur bewährt. Wenn fremde, ansonsten nicht am kontaminierten Standort lebende Bakterientypen eingesetzt wurden, konnten sich diese gegen die spezifisch angepasste einheimische Flora meist nicht durchsetzen (vergleiche **Veröffentlichung III** von Zhu et al. "Literature review on the use of commercial bioremediation agents for cleanup of oil-contaminated estuarine environments" Bericht EPA/600/R-04/075 July 2004). Dabei wurde in der Regel auch nur ein einzelner kohlenwasserstoffabbauender Bakterientyp, beispielsweise aus der Gattung *Pseudomonas* (DE 38 11 856 C2), *Bacillus* (DE 196 52 580 A1) oder *Acetobacter* (DE 44 43 266 A1) verwendet. Es kam im besten Fall zu einer kurzzeitigen Beschleunigung des Abbaus. Nach ca. 2 Wochen waren die eingeimpften Organismen von der einheimischen Flora überwuchert.

Der Einsatz von natürlichen Bakteriengemischen wird in den japanischen Abstracts JP 2004181314 A und JP 200607542 A für die Sanierung von Böden beschrieben. Im Unterschied zum Meereis sind Böden aber relativ homogene und stabile Habitate, in denen Organismen keinem so starken physiko/chemischen Gradienten unterliegen wie im Meereis, außerdem können eingebrachte Bakterien nicht so leicht ausgewaschen werden. Ferner ist aus der EP 0 859 747 B1 ein Verfahren zum aeroben biologischen Abbau schwer wasserlöslicher Stoffe bekannt, das eine Kultur eines thermophilen Mikroorganismus IHI-91 (DSM 10561) verwendet. Dieser Stamm hat eine große Ähnlichkeit wiederum zu der Gattung *Bacillus.* Zum Schutzumfang des europäischen Patents gehören auch der Mikroorganismus IHI-91 selbst und die daraus erhältliche Enzymzusammensetzung zum Zweck des biologischen Abbaus. Die oben bereits genannte DE 196 52 580 A1 nennt zwar ein "bakterielles Gemisch" zur Regenerierung von durch Erdöl und/oder Ölprodukte verunreinigten Böden und Gewässern. Neben der Bakteriengattung Bacillus wird noch ein unspezifisches, durch Hydrolyse von Mikrobiomasse (Abfall) Gemisch entstandenes "Biosurfaktant BS-4" eingesetzt, von dem nicht ersichtlich ist, ob darin weitere Bakterientypen enthalten sind. Aufgrund der starken chemischen Hydrolyse ist aber davon auszugehen, dass es sich vielmehr um ein Stoffgemisch unter Beteiligung einer einzigen Bakteriengattung handelt. Aus der DE 199 54 643 A1 schließlich ist die Herstellung und Anwendung eines Ölbindemittels zum Entfernen von Ölen und Fetten aller Art von wässrigen und festen Oberflächen bekannt. Es werden Mikroorganismen an faserbildende Proteine in Granulatform immobilisiert. Hierbei kann es sich um *Pseudomonas putida, Pseudomonas spec., Acinetobacter calcoaaceticus, Nocardia spec., Corynebacterium spec., Candida lipolytica, Candida tropicalis, Rhodopseudomonas palistris* oder *Rhodococcus spec.* handeln. Ob Bakteriengemische immobilisiert werden, wird nicht ausgeführt. Ferner werden keine speziellen Angaben für die Auswahl der genannten Mikroorganismen gemacht. Alle genannten Ausführungsbeispiele umfassen keine Immobilisierung von Mikroorganismen.

Aus der **Veröffentlichung** "Degradation of crude oil by an arctic microbial consortium" (U.Deppe at. al,. Extremophiles; Life under extreme conditions, Springer Verlag, TO, Bd. 9, Nr.6, 1.Dez. 2005, Seiten 461-470) ist eine flüssige Anreicherungskultur mit +4°C kaltem Meerwasser bekannt, die mit Öl versetzt wird und insgesamt in dieser einmaligen Zusammenstellung Öl abbaut. Es werden jedoch keine Einzelisolate, die ein unterschiedliches Abbauvermögen haben, bereitgestellt, sondern ein komplexes Konsortium erhalten. Die Bioverfügbarkeit von Öl für Mikroben ist bei +4°C eine völlig andere als unterhalb des Gefrierpunkts.

Allen zuvor genannten Druckschriften ist gemeinsam, dass sie einen biologischen Ölabbau durch Bakterien verschiedener Gattungen und Spezies nur unter normalen oder sogar erhöhten (thermophile Bakterien) Umgebungstemperaturen, nicht aber bei tiefen Temperaturen (per Definition sind kälteangepassten Bakterien Organismen, deren Wachtumstemperaturminimum bei 0°C oder tiefer liegt, wobei die Gruppe der kälteangepassten Organismen weiter in die so genannten "psychrophilen Organismen" mit einem Wachstums-Temperaturmaximum unter 20°C und in die "psychrotoleranten Organismen" mit einem Wachstums-Temperaturmaximum über 20°C unterteilt wird) beschreiben. Aus der DE 10 2005 028 295 A1 ist lediglich die Verwendung von psychrophilen Proteasen aus der Gattung *Shewanella* zur Entfernung von Biofilmen von harten Oberflächen bekannt. Unter den genannten Anwendungen befinden sich aber keine im tiefen Temperaturbereich um oder unter dem Gefrierpunkt. Vielmehr ist davon auszugehen, dass durch die Anwendung der psychrophilen Proteasen eine Entfernung von Schmutz und Biofilmen bei bisher 37°C in einen Bereich zwischen 15-20°C verlagert werden soll (siehe Absätze [006] bis [008]). Eine Entfernung von Biofilmen unter Meereisbedingungen ist nicht offenbart.

Das Meereis mit seinen extrem niedrigen Temperaturen und stark variierenden Salzgehalten ist vielmehr ein lebensfeindlicher Standort, der dem Fachmann für Bioremediationsverfahren ungeeignet scheint. Das bisher einzige Bioremediationsexperiment im Meereis wurde von Delille et al. (Veröffentlichung IV: "Seasonal Variation of Bacteria in Sea Ice Contaminated by Diesel Fuel and Dispersed Crude Oil", Microb. Ecol. (1997) 33 pp 97-105) veröffentlicht. Antarktisches Meereis wurde mit arabischem Rohöl bzw. Dieselöl kontaminiert und ein Teil der Versuchsfelder mit dem organischen Nährstoffkomplex INIPOL EAP 22 gedüngt. In den gedüngten, allerdings auch in den ungedüngten mit Öl kontaminierten Versuchsfeldern wurde eine Zunahme von Mikroorganismen im Vergleich zum Kontrollfeld beobachtet. Ungeprüft blieb, ob es zu einem Ölabbau kam, wie stark dieser war und welche Komponenten verschwanden. Die nach der Ausbringung von Öl und Nährstoffen vorhandenen Organismen wurden nicht isoliert und nicht charakterisiert. Allerdings deutet die Zunahme der Mikroorganismen daraufhin, dass das Öl auf Teile der Meereisgemeinschaft zumindest nicht toxisch wirkt.

Wie bereits erwähnt, stehen dem Einsatz von Bioremediationsverfahren im Meereis die dort herrschenden lebensfeindlichen Bedingungen, d.h. sehr kalte Temperatur mit zum Teil sehr hohen Salinitäten, sowie die starken Variationen der Temperatur- und Salzbedingungen über das Eisschollenprofil und über die Jahreszeit entgegen. Mikrobielles Wachstum und Aktivitäten sind auch bei kälteangepassten Bakterien im Temperaturbereich von ca. -5°C bis -3°C, wie sie im unteren Eisbereich (Wasser/Eis Grenzbereich) herrschen, gering und im Bereich der Eisoberfläche (Temperaturspanne von ca. +1°C im Sommer bis unter -20°C im Winter) sogar nur auf die kurze Sommersaison begrenzt. Das Meereis ist von einer spezifischen, eng an den Standort angepassten Mikrobengemeinschaft besiedelt. Da polares Meereis bisher kaum mit Erdöl konfrontiert wurde, ist das Auftreten und eine schnelle Entwicklung einer natürlichen kohlenwasserstoffabbauenden Flora im Fall einer Ölkontamination daher bislang für den Fachmann wenig wahrscheinlich. Einerseits sprechen diese Fakten gegen den Einsatz von Bioremediationsverfahren im Eis, andererseits bieten aber biologische Verfahren im Vergleich zu den physikalisch/chemischen Verfahren gerade im Meereis viele Vorteile. Sie könnten quasi bei jeder Eissituation und jedem Ölverschmutzungsszenario angewendet werden, d.h., ob das Öl sich auf dem Eis, unter dem Eis oder im freien Wasser befindet. Weiterhin müssten einmal ausgebrachte Nährstoffe und Organismen nicht weiter betreut werden und schließlich müsste man in dem schwer zugänglichen Gebiet nicht für die Bekämpfungsmaßnahmen Vorort bleiben, sondern könnte das System nach der Präparation sich selbst überlassen.

Konkrete Bioremediationsverfahren zum Ölabbau in Meereisgebieten werden im Stand der Technik nicht vorgeschlagen (vergleiche Veröffentlichung I, Seite 88, Kap. 5.6). In der **Veröffentlichung V** von B.Gerdes et al. (WP4 Environmental Protection, "Biological degradation of crude oil in Artic sea ice", ARCOP Workshop 8, 19.-20. Oct. 2005, St.Petersburg) wird jedoch die Möglichkeit eines Bioremediationsverfahrens zur Beschleunigung natürlicher aerober Abbauprozesse durch Bioaugmentation von exogenen Mikroben und/oder durch die Zugabe von Nährstoffen und/oder Sauerstoff in polaren Eisregionen aufgezeigt. Als limitierende Faktoren für den Abbau werden Nährstoffe, Verfügbarkeit von Sauerstoff, Temperatur, Spurenelemente wie Eisen, Salinität und pH-Wert, Löslichkeit und Tröpfchengröße des Öls genannt. Es zeigte sich jedoch, dass im arktischen Winter bei Temperaturen von -3°C kein signifikanter Ölabbau weder durch Düngung mit Nährstoffen noch durch Inokulation stattfindet, wobei sich bei 0°C in anorganisch gedüngten Schmelzwasserproben eine signifikante Veränderung der Bakterienvielfalt zeigte.

Der genannten **Veröffentlichung V,** von der die vorliegende Erfindung als nächstliegendem Stand der Technik ausgeht, ist also zu entnehmen, dass die Durchführung eines Bioremediationsverfahrens zum beschleunigten biologischen Abbau von Petroleum-Kohlenwasserstoffen in den polaren Eisregionen durch Bioaugmentation von kohlenwasserstoffabbauenden Bakterien unter Zugabe von Nährstoffen bei Temperaturen oberhalb des Gefrierpunkts möglich ist. Unterhalb des Gefrierpunktes scheint die Biodegradation zu stoppen. Über Art und Weise des Bioremediationsverfahrens und über geeignete Bakteriengemische als Mittel zur Verfahrensdurchführung werden jedoch keine weiteren Aussagen gemacht.

### AUFGABENSTELLUNG

Die **Aufgabe** für die vorliegende Erfindung ist daher darin zu sehen, das zuvor beschriebene gattungsgemäße Bioremediationsverfahrens so weiterzubilden, dass ein biologischer Abbau von Kohlenwasserstoffen, insbesondere in Form von Ölverschmutzungen geringeren Ausmaßes, unter den in den polaren Eisregionen auftretenden Bedingungen, insbesondere auch unterhalb des Gefrierpunkts, und an verschiedenen Orten (regional und lokal) der auftretenden Verschmutzung zuverlässig, möglichst schnell und in ausreichendem Ausmaß stattfindet. Dabei soll das weitergebildete Bioremediationsverfahren einfach und kostengünstig in seiner Durchführung sein und ein Minimum an Personenaufwand in den extremen Eisregionen ermöglichen. Gleichzeitig sollen bevorzugte Mittel zur Durchführung des Bioremediationsverfahrens zur Verfügung gestellt werden, durch deren Einsatz die Effektivität des Bioremediationsverfahrens besonders gesteigert werden kann. Die erfindungsgemäße **Lösung** für diese Aufgaben ist dem Verfahrensanspruch und den nebengeordneten Mittelansprüchen zu entnehmen. Vorteilhafte Weiterbildungen werden in den jeweiligen Unteransprüchen aufgezeigt und im Folgenden im Zusammenhang mit der Erfindung näher erläutert.

Mit dem Verfahrensanspruch wird ein Bioremediationsverfahren beansprucht, das gekennzeichnet ist durch
eine Inkontaktbringung der abzubauenden Petroleum-Kohlenwasserstoffe mit einem Inokulum aus zumindest nachfolgenden Komponenten:
- einem Bakteriengemisch aus unterschiedlichen kälteangepassten, autochthonen Bakterienstämmen, die gewinnbar sind durch
   - Ansetzen eines labortechnischen Mesokosmos mit Meereis von einem aktuellen oder potenziellen Abbauort,
   - künstliche Kontamination des Mesokosmos mit Rohöl,
   - Versehen des Mesokosmos mit Nährstoffen,
   - Inkubation des Mesokosmos bei -3°C über einen Zeitraum von 12 bis 36 Monaten in der Form, dass die Eis-Wassersituation stabil bleibt,
   - Isolation der dominanten Bakterienstämme und
   - Auswahl der isolierten Bakterienstämme danach, dass sie
      - auch bei einer Umgebungstemperatur -3°C aktiv sind,
      - einen unterschiedlichen Temperaturtoleranzbereich aufweisen,
      - einen unterschiedlichen Salztoleranzbereich besitzen,
      - ein unterschiedliches Abbauspektrum haben und
      - ein unterschiedliches Potenzial aufweisen Öl zu emulgieren,
- den Nährstoffen und
- einem umweltfreundlichen Trägermaterial, an dem zumindest die Bakterien der Bakterienstämme immobilisiert sind.

Bei dem erfindungsgemäßen Bioremediationsverfahren wird erfindungswesentlich ein Gemisch aus mehreren verschiedenen kälteangepassten, autochthonen Bakterienstämmen eingesetzt. Dabei weist jeder eingesetzte Bakterienstamm einen anderen Temperatur- und Salztoleranzbereich, ein anderes Abbauspektrum, eine andere Abbauaktivität sowie eine andere Fähigkeit zur Ölemulgierung auf. Durch diese Kombination unterschiedlicher Typen von Bakterienstämmen entsteht ein sich ergänzendes, in der Abbauwirkung äußerst breitbandiges Bakteriengemisch, welches unter den unterschiedlichsten Meereisbedingungen und Kontaminationsszenarien den biologischen Abbau einer breiten Palette von Petroleum-Kohlenwasserstoffen sicher bewerkstelligen kann. Dabei variieren die eingesetzten kälteangepassten Bakterienstämme in ihrem Temperatur und Salztoleranzbereich, um dem Bioremediationsverfahren bei allen im natürlichen Habitat auftretenden Temperaturen Effektivität zu geben. Allen Bakterienstämmen im Gemisch ist jedoch gemeinsam, dass sie auch noch eine Aktivität (Abbau oder Ausbildung von Emulgatoren) bei -3°C zeigen. Ein Ölabbau bei dieser tiefen Temperatur ist für den Fachmann überraschend und noch nirgends im Stand der Technik dokumentiert. Aus diesem Grund wurde bislang der Einsatz eines Bioremediationsverfahren in polaren Meereisregionen für uneffektiv erachtet.

Das bei dem erfindungsgemäßen Bioremediationsverfahren eingesetzte Bakteriengemisch hat nicht nur ein breiteres Abbauspektrum als die Einzelorganismen, sondern es reagiert auch besser auf Schwankungen im Ökosystem, was bei den sehr variablen Bedingungen im Eis wichtig ist. Durch die breitbandige Aktivität des Bakteriengemisches kann eine Beseitigung von Öl-(Rest-) Verschmutzungen kleineren Ausmaßes sowohl im Meereis, als auch in Schmelztümpeln und im angrenzenden Wasser durch Bioaugmentation mit dem Ausbringen von nur einem einzigen Inokulum gewährleistet werden. Dabei wird das Bakteriengemisch in hohen Konzentrationen eingesetzt, um die langsame Regeneration und Reproduktion im Eis zu kompensieren.

Erfindungsgemäß gewinnbar ist das Bakteriengemisch durch eine Kombination von Bakterienstämmen, die sich in Mesokosmosexperimenten über einen Zeitraum von 12 bis 36 Monaten anreichern und anschließend isoliert werden. Die Mesokosmen werden mit Eis von einem möglichen Abbauort angesetzt, künstlich mit Rohöl kontaminiert, mit Nährstoffen versehen und bei -3°C so inkubiert, dass die Eis-Wassersituation stabil bleibt. Dabei ist es keineswegs erforderlich, dass für jeden Einsatzfall des Bioremediationsverfahrens spezielle Bakterienstämme nach dem zuvor angegeben Verfahren isoliert werden. Vielmehr kann in beispielhaften Mesokosmosexperimenten eine ganze Vielzahl geeigneter Bakterienstämme gewonnen und archiviert werden. Für den aktuellen Einsatzfall für das Bioremediationsverfahrens können dann entsprechend der Einsatzbedingungen, vor allem des Einsatzortes individuell die passenden Bakterienstämme im Bakteriengemisch kombiniert werden. So ist durchaus eine schnelle Reaktion auf Ölverschmutzungen durch Einsatz des Bioremediationsverfahrens nach der Erfindung möglich. Die auf diese Weise gewonnenen Bakterienstämme ergänzen sich in den physiologischen Eigenschaften und sind in der Mischung in der Lage, unter den für Meereis typischen, stark variierenden Salz- und Temperaturbedingungen Öl abzubauen. Durch den Zusatz anderer Öl abbauender Bakterienstämme und/oder durch die Ausbringung von Kohlenwasserstoff abbauenden Enzymen aus diesen kälteangepassten Mikroorganismen kann das Bioremediationsverfahren nach der Erfindung noch effektiver, flexibler und breiter einsetzbar gemacht werden.

Durch die erfindungsgemäße Art der Gewinnbarkeit des Bakteriengemisches wird sichergestellt, dass grundsätzlich autochthone, das heißt, im natürlichen Habitat vorkommende Bakterienstämme eingesetzt werden. Damit eignet sich das Bioremediationsverfahren nach der Erfindung neben der Beseitigung von kleineren Ölverschmutzungen insbesondere für Restverschmutzungen nach dem Einsatz von mechanischen Ölbekämpfungsmaßnahmen oder "in situ Verbrennungen" in deren Folge es zu einer Zerstörung der natürlichen Meereisflora kommt, die durch die Beimpfung mit dem Öl abbauenden Bakteriengemisch kompensiert wird. Durch die Verwendung autochthoner Meereisbakterien kommt es zu einem Wiederaufbau der natürlichen Mikroflora nach einer erfolgten mechanischen und/oder physikalischen Behandlung des Meereises. Damit ist das Bioremediationsverfahren nach der Erfindung in höchstem Maße umweltfreundlich. Es entfernt Ölverschmutzungen und regeneriert gleichzeitig die natürliche Mikroflora. Außerdem verwendet es umweltfreundliche Trägermaterialien.

Das Bakteriengemisch als Mittel zur Durchführung des Bioremediationsverfahrens ist erfindungsgemäß gekennzeichnet durch eine Kombination von Bakterienstämmen, die gewinnbar sind durch eine Anreicherung in einem labortechnischen Mesokosmosaufbau über einen Zeitraum von 12 bis 36 Monaten, anschließende Isolation der dominanten Bakterienstämme und Auswahl der isolierten Bakterienstämme danach, dass sie auch bei einer Umgebungstemperatur -3°C aktiv sind, einen unterschiedlichen Temperaturtoleranzbereich aufweisen, einen unterschiedlichen Salztoleranzbereich besitzen, ein unterschiedliches Abbauspektrum haben und ein unterschiedliches Potenzial aufweisen Öl zu emulgieren, wobei der Mesokosmos mit Meereis von einem aktuellen oder potenziellen Abbauort angesetzt, künstlich mit Rohöl kontaminiert, mit Nährstoffen versehen und bei -3°C so inkubiert wird, dass die Eis-Wassersituation stabil bleibt.

Das Bioremediationsverfahren nach der Erfindung mit den bei der DSMZ - Deutsche Sammlung von Mikroorganismen und Zellkulturen GmbH, Inhoffenstraße 7B, 38124 Braunschweig, Deutschland - hinterlegten Bakterienstämmen ist besonders vorteilhaft für Kontaminationen im arktischen Meereis und mit Öl, welches reich an aliphatischen Verbindungen ist. Es handelt sich um neue gegenüber bekannten Bakterienstämmen taxonomisch abgegrenzte Bakterienstämme mit deutlich anderer Physiologie. Das Vorkommen dieser spezifischen Mikroorganismen wurde bis dato im Meereis nicht vermutet. Das Verfahren ist aber nicht auf dieses Bakteriengemisch beschränkt, sondern kann durch den Zusatz von anderen, nach dem erfindungsgemäßen Verfahren gewinnbaren Bakterienstämmen, die beispielsweise mehr auf den Abbau aromatischer Kohlenwasserstoffe (polyaromatische Kohlenwasserstoffe) oder Schweröle spezialisiert sind, erweitert werden. Auch bei Kontaminationen im antarktischen Bereich, wo laut dem Antarktis-Vertragssystem nur autochthon antarktische Bakterien verwendet werden dürfen, können entsprechend dem Verfahren gewonnene, autochthone Bakterienstämme eingesetzt werden.

Das Enzymgemisch als Mittel zur Durchführung des Bioremediationsverfahrens ist erfindungsgemäß gekennzeichnet durch eine Kombination unterschiedlicher Enzyme oder Enzymzusammensetzungen, die gewinnbar sind aus den Bakterienstämmen zur Gewinnung der Bakteriengemische, wobei die Bakterienstämme danach ausgewählt sind, dass sie auch bei einer Umgebungstemperatur -3°C aktiv sind, einen unterschiedlichen Temperaturtoleranzbereich aufweisen, einen unterschiedlichen Salztoleranzbereich besitzen, ein unterschiedliches Abbauspektrum haben und ein unterschiedliches Potenzial aufweisen Öl zu emulgieren.

Durch die Verwendung von kälteangepassten Enzymzusammensetzungen aus den isolierten Bakterienstämmen wird die Anwendung des Bioremediationsverfahrens nach der Erfindung noch breiter, da physiko/chemische Bedingungen dann keinen so starken Einfluss mehr auf die Abbauaktivität haben wie bei intakten Mikroorganismen. Das Verfahren kann bei Verwendung der kälteangepassten Enzymzusammensetzungen in allen kalten Gebieten limnisch wie marin, pelagisch wie benthisch angewendet werden, d.h. in allen antarktischen Habitaten und auch in arktischen Tiefseesedimenten, wo insbesondere spezifische, an den hohen hydrostatischen Druck angepasste Bakterien aktiv sind, und sogar in Permafrostböden.

Durch die spezielle Zusammensetzung des Inokulums kann das Bioremediationsverfahren nach der Erfindung individuell auf spezifische Bedingungen eingestellt werden. Dabei kann das Verfahren einmal angewendet und das Inokulum einmal nach dem Unfall ausgebracht werden. Es arbeitet dann ohne Personalerfordernis in situ, auch wenn beispielsweise beimpfte Eisschollen räumlich verfrachtet werden. Es können aber auch Nachregulierungen durch Nachimpfen von gleichen oder anderen Mikroorganismen (z.B. Abbauern von polyaliphatischen Kohlenwasserstoffen) oder zusätzliche Nährstoffgaben erfolgen. Das Nachimpfen ist abhängig von der Qualität und Quantität der abzubauenden Kohlenwasserstoffe sowie von den Umweltbedingungen und muss von Fall zu Fall unterschiedlich gehandhabt werden.

Weitere vorteilhafte Weiterbildungen des Bioremediationsverfahrens nach der Erfindung sind dem speziellen Beschreibungsteil zu entnehmen. Speziell genannt seien an dieser Stelle die Immobilisierung des Inokulums an spezielle Trägermaterialien und die Art der Ausbringung des Inokulums im Einsatzfall. Das komplexe Bakteriengemisch wird an umweltfreundliches Trägermaterial immobilisiert, welches mit bevorzugt anorganischen Nährstoffen versehen wird oder selbst als Nährstoff dient. Entsprechend der Kontamination und der Eisverhältnisse kann schwimmendes oder nicht schwimmendes, hydrophiles oder hydrophobes Trägermaterial sowie auch ölbindendes Trägermaterial verwendet werden. Dieses kann direkt auf das Meereis, auf das angrenzende Wasser oder auch unter das Meereis verbracht werden. Die Ausbringung des Inokulums auf die Eisoberfläche kann per Schiff, per Löschkanone oder per Flugzeug erfolgen. Eine Ausbringung unter dem Eis durch Einleiten oder Pumpen des Inokulums in die Wassersäule unter dem Eis ist ebenfalls möglich. Dadurch ergibt sich kein begrenzter Einsatz des Bioremediationsverfahrens nach der Erfindung wie bei den mechanischen (nur bei kleinen Schollen) und physikalischen Methoden (nur bei dichter Eisdecke oder fast offenem Wasser). Bei Einsatz von Helikoptern kann die Ausbringung des Inokulums auch unter schwierigsten Eisbedingungen erfolgen, beispielsweise bei dichter Eisbedeckung und bei Eisrückenbildung.

### AUSFÜHRUNGSBEISPIEL

Ausbildungsformen des beanspruchten Bioremediationsverfahren zum beschleunigten biologischen Abbau von Petroleum-Kohlenwasserstoffen in den polaren Eisregionen und der beanspruchten Bakteriengemische als Mittel zur Verfahrensdurchführung werden nachfolgend näher erläutert.

Dabei zeigt die **Tabelle 1** eine Auswahl geeigneter Bakterienstämme für bevorzugte Bakteriengemische, wobei die ersten sechs Bakterienstämme zu einem basisbildenden Bakteriengemisch zusammengeführt werden können, das durch Zusatz der weiteren fünf genannten Bakterienstämme optional an den speziellen Einsatzfall angepasst und dadurch in seiner Abbaueffektivität noch verbessert werden kann.

Die **Tabelle 2** zeigt den Abbau von aliphatischen Komponenten von Rohöl (0,2%) in Spaltenwasser (gap water) und in einem arktischen Meereis-Mesokosmosaufbau durch die bevorzugten Bakteriengemische.

Um das Bioremediationsverfahren nach der Erfindung anwenden zu können, sind mehrere, nachfolgend aufgeführte Verfahrensschritte grundlegender, vorbereitender und auszuführender Art abzuarbeiten. Dabei können die grundlegenden und vorbereitenden Verfahrensschritte durchaus entfallen, wenn für einen speziellen Einsatzfall bereits geeignete isolierte Bakterienstämme oder Inokula vorliegen.
- Anreicherung und Isolation von speziellen Bakterienstämmen, die unter stabilen Meereisbedingungen, d.h. noch bei Temperaturen von -3°C , Erdöl emulgieren und in verhältnismäßig kurzer Zeit Komponenten davon abbauen
- Kombination von unterschiedlichen Typen der gewonnenen Meereisbakterienstämme in einer solchen Weise, dass ein sich ergänzendes Bakteriengemisch entsteht, welches unter den unterschiedlichsten Meereisbedingungen und Kontaminationsszenarien den bestmöglich beschleunigten Abbau einer möglichst breiten Palette von Rohölkomponenten bewerkstelligt
- Produktion einer hinreichend aktiven Biomasse aus den ausgesuchten Bakterienstämmen
- Bildung eines Inokulums durch Immobilisierung der aktiven Biomasse zusammen mit geeigneten Nährstoffen an einem Trägermaterial
- Ausbringen der Inokulums zur Inkontaktbringung mit den abzubauenden Petroleum-Kohlenwasserstoffen.

Zur Gewinnung geeigneter Bakterienstämme wurden beispielsweise bei der Anmelderin unterschiedliche Eiskerne von mehrjährigem arktischem Meereis zwischen September und Oktober (Sommer) sowie zwischen April und Mai (Winter) im Bereich nordöstlich von Spitzbergen (79° bis 81 °N und 5° bis 12° Ost) gewonnen. Ebenso wurde antarktisches Meereis aus der Bellinghausen See (66 -71°CS und 275 - 290°W) im April bis Mai (Herbst) beprobt. Im Heimatlabor wurden zirka fünf Liter des unter sterilen Bedingungen kleingeschlagenen Eises zusammen mit 10 Litern sterilem polarem Wasser in sterile Glasbehälter, gefüllt. Auf das schwimmende Eis wurde frisches bzw. abgelagertes Rohöl (0,1%) verteilt. Die unterschiedlichen Mesokosmen erhielten verschiedene Nährstoffe:
- Inipol MS3000
- Fischmehl
- anorganische Nährstoffe, bestehend aus: Na₂HPO₄ 0,065gl⁻¹, NaNO₃ 0.75gl⁻¹, Spuren von FePO₄).

Die Experimente wurden über einen Zeitraum von 36 Monaten bei -3°C inkubiert. Parallel wurden Experimente mit geschmolzenem Eis bei +4°C bebrütet. Nach eineinhalb Jahren wurde erstmals Probenmaterial entnommen, seriell verdünnt und 100 µl der einzelnen Verdünnungsstufen auf Minimal Agar ausgestrichen. Auf den Agarplatten war zuvor 20µl Rohöl verteilt worden. Die Platten wurden bei +4°C inkubiert, bis Kolonien sichtbar wurden. Diese wurden gepickt und wiederholt ausgestrichen, bis Reinkulturen vorlagen. Die unterschiedlichen Stämme wurden mit Hilfe von ARDRA (Amplified rDNA Restriction Analysis) taxonomisch gruppiert und die Sequenzen des 16S rRNA Gens von den jeweiligen Gruppen bestimmt. Die Typen wurden weiterhin auf physiologische Charakteristika sowie Temperaturverhalten und Kohlenwasserstoff-Abbaupotenzial untersucht. Überraschenderweise konnten neue, kälteangepasste Bakterientypen entdeckt werden, die bei -3°C Kohlenwasserstoffe abbauen. Die Sequenzähnlichkeit des 16S rRNA Gens der Stämme zu den nächstverwandten Typstämmen wird in der **Tabelle 1** angegeben.

Sechs unterschiedliche Bakterientypen, die sich von bisher bekannten Mikroorganismen unterscheiden, können bevorzugt als Gemisch eingesetzt werden. Die Zugabe der angegebenen Bakterienstämme erfolgt bevorzugt in gleichen Anteilen. Je nach Einsatzfall werden sich dann die am besten arbeitenden Bakterienstämme dominant entwickeln. Weiterhin wurde überraschenderweise durch die Zusammenstellung von unterschiedlichen Bakterientypen mit unterschiedlicher Temperatur- und Salztoleranz sowie unterschiedlichem Abbaupotenzial letztendlich ein Bakteriengemisch erhalten, welches insgesamt in der Lage ist, sich bei unterschiedlichen Eisbedingungen zu entwickeln, Erdöl zu emulgieren und in akzeptabler Zeit abzubauen.

Die in **Tabelle 1** aufgezeigten Bakterienstämme sind bei der DSMZ hinterlegt. Sie können als wachsende Stammkultur in Röhrchen in 10 ml Minimalmedium (KNO₃ 0,75 g, NH₄Cl 0,75 g, Hefeextrakt 0,1 mg (Difco), Spuren von FePO₄. in 1 Liter 50% arktisches Seewasser) plus 20 µl Rohöl für ca. ein halbes Jahr bei 1°C aufbewahrt oder konserviert werden (z.B. MAST Cryobank System, - 80°C oder Gefriertrocknung). Für den Einsatz bzw. zur Beimpfung der Anzuchten müssen die Stämme aus der Konserve geholt bzw. die Stammkulturen verwendet werden. Wenn konserviertes Material eingesetzt wird, muss dieses zunächst in Seewasser-Nährmedium (5g Bacto Pepton (Difco), 1 g Hefeextrakt (Difco) und Spuren von FePO₄ in 1 Liter 50% arktisches Seewasser oder Marine Broth 2216 (Difco)) bei 4°C rekultiviert werden, bei den Bakterienstämmen GH-10 (DSM 18952) und GH-11 (DSM 18953) sollte 10fach verdünntes Seewasser-Nährmedium genommen werden und 0,1% steriles Rohöl zugesetzt werden. Sobald hinreichendes Wachstum vorhanden ist (abhängig vom Stamm dauert dies ca. 2 Tage bis 1 Woche) werden mit diesen Kulturen größere Volumina (500ml bis ca. 10I) von Minimalmedium (KNO₃ 0,75 g, NH₄Cl 0,75 g, Hefeextrakt 0,1 mg (Difco), Spuren von FePO₄, in 1 Liter 50% arktisches Seewasser) plus 0,2% (v/v) Öl beimpft. Die Stämme werden separat unter Schütteln (100 rpm) bei 4°C angezogen bis zu einer Zelldicht von mindestens 1x10⁹ Zellen pro ml (ca. 1 Woche). Durch den Rohölzusatz stellen sich die Stämme auf den Kohlenwasserstoffabbau ein.

Bei dem Bioremediationsverfahren nach der Erfindung müssen die verwendeten Mikroorganismen und/oder die daraus gewinnbaren Enzymzusammensetzungen mit Hilfe von Trägermaterial immobilisiert werden und an den Kontaminationsort verbracht werden. Für einen optimalen Kohlenwasserstoffabbau ist bei den Mikroorganismen die Versorgung mit Stickstoff- und Phosphatquellen notwendig. Damit Nährstoffe und Mikroorganismen zusammen an den Ort der Kontamination gelangen und verbleiben, müssen geeignete umweltfreundliche Trägermaterialien eingesetzt werden. Bei der Auswahl des Trägermaterials werden die unterschiedlichen Meereiskontaminations-Szenarien berücksichtigt. Es wird hydrophobes sowie hydrophiles Trägermaterial eingesetzt, welches schwimmfähig bzw. nicht schwimmfähig ist. Es empfiehlt sich, das Bakteriengemisch nicht nur auf einem, sondern mindestens auf zwei verschiedenen Trägermaterialien immobilisiert auszubringen, damit unterschiedliche Einsatzorte erreicht werden können. Folgende Trägermaterialien können bevorzugt eingesetzt werden:

### Trägermaterial 1:

Auf der Basis des in der US-PS 5 954 868 beschriebenen partikulären Materials besteht das Trägermaterial 1 aus einem Kern von Nährstoffen, z.B. NH₄H₂PO₄ und FeSO₄ im Verhältnis 90:8:2 von Ammonium/ Phosphat/ Eisen, und ist umhüllt von oleophilen Komponenten, wie gesättigten und ungesättigten Fettsäuren (z.B. Ölsäure, Stearinsäure oder/und Palmitinsäure). Das Trägermaterial 1 ist oleophil und schwimmt, die organische Komponente wird mit einer anorganischen kombiniert, es hinterlässt nach dem Einsatz keinerlei Rückstände.

### Trägermaterial 2:

Basierend auf der "Bio-Sep-Technologie" (University Tulsa, OK 74104, USA), besteht das Trägermaterial 2 aus Kugeln von 3-4 mm Durchmesser, die aus 25% Aramid-Polymer (Nomex) und 75% Aktivkohle (PAC) hergestellt werden und eine Porosität von 75% aufweisen. Der mittlere Porendurchmesser ist 1,9 µm. Die Kugeln sind mit einer Ultrafiltrationsmembran ähnlichen Membran umgeben. Die Nährstoffe werden von den Kugeln aufgesogen, die Organismen siedeln sich im Inneren der Kugeln an. Die Kugeln wurden bisher für Grundwassersanierung eingesetzt. Das Trägermaterial 2 ist oleophil und schwimmt, es können organische wie anorganische Nährstoffe verwendet werden. Nachteilig ist nur, dass das Aramid ein Kunststoff ist und nach dem Einsatz im Ökosystem verbleiben wird.

### Trägermaterial 3:

Basierend auf der deutschen Offenlegung DE 199 54 643 A1 basiert das Trägermaterial 3 auf einem Ölbindemittel aus faserbildenden Proteinen. Das Material wird mit Nährstoffen beladen, es absorbiert Öl und ist schwimmfähig und daher bevorzugt anzuwenden, wenn freies Wasser vorhanden ist bzw. Öl unter das Eis gelangt ist.

### Trägermaterial 4:

Das Trägermaterial 4 basiert auf Fischmehl, das mit einem Polysorbat 80 (Polyoxyethylensorbitanmonooleat, kommerzieller Name auch Tween® 80) versetzt ist, welches als nichtionisches Tensid in Kosmetika, Arzneimitteln, Futtermitteln und besonders als Emulgator in Lebensmitteln verwendet wird. Das Polysorbat wirkt emulgierend, sodass sich das Trägermaterial an die Ölkontamination ansetzt. Dieses Trägermaterial 4 ist besonders für Kontaminationen an der Meereisoberfläche, aber auch im Inneren des Meereises geeignet.

### Trägermaterial 5:

Das Trägermaterial 5 basiert auf Sägespänen (0,3- 1 mm Partikeldurchmesser), die mit Nährstoffen(NH₄H₂PO₄ 0,065gl⁻¹, NaNO₃ 0,75 gl⁻¹ und Spuren von FeCl₃) getränkt sind. Die Sägespäne sind hydrophil und schwimmen daher nicht auf Wasser, daher ist dieses Trägermaterial 5 besonders für kleinere Kontaminationen an der Oberfläche von Eis, aber auch im Inneren des Meereises geeignet, da die Sägespäne in tiefere Eisschichten absinken können.

Für die Immobilisierung der Zellen werden die einzelnen großvolumigen Anzuchten (in Minimalmedium mit Öl) von den verschiedenen Bakterienstämmen in einem sterilen Gefäß zusammengeführt und gemischt. Das Gemisch wird danach wieder auf so viele Gefäße aufgeteilt wie unterschiedliche Trägerstoffe verwendet werden sollen. Pro 1 Liter Gemisch wird 20 g steriles Trägermaterial zugesetzt und der Ansatz nochmals 3 Tage bei 4°C geschwenkt (90 rpm), um den Zellen die Möglichkeit zur Anheftung an das Trägermaterial zu geben. Das Trägermaterial kann danach je nach Ausbringungsverfahren weitgehend von der Nährlösung getrennt werden. Das Material kann so auf dem Eis verteilt werden. Es kann gesprüht, gespritzt, gegossen oder auch unters Eis gepumpt werden, je nachdem, was für Gerätschaften zur Verfügung stehen und um welche Art Einsatzfall es sich handelt. Beim Versprühen empfiehlt es sich, das Trägermaterial nicht von der Nährlösung zu trennen, sondern diese mitzuversprühen. Die einzelnen Ausbringungsverfahren müssen auf die Trägermaterialien abgestimmt werden. Insgesamt empfiehlt es sich zum Schutz der kälteangepassten Mikroorganismen, das Inokulum niemals über Kühlschranktemperatur (+7°C) zu lagern oder einzusetzen. Oberhalb dieser Temperatur könnten die Mikroorganismen beschädigt werden und beispielsweise ihre Abbauaktivität signifikant reduzieren.

Bei einem großflächigen Ölunfall werden große Mengen an Biomasse ausgebracht, dafür erfolgt zunächst ein Upscaling der Anzucht. Um bei einem akuten Ölunfall schnell reagieren zu können, werden Zellmassen für einen Einsatz gefriergetrocknet oder eingefroren mit Gefrierschutz bereitgehalten. Dabei ist zu berücksichtigen, dass die Konservierungsverfahren einen Teil der Zellen schädigen. Die konservierten Zellen werden daher vor dem Ausbringen in einer kurzen, dreitägigen Inkubationsphase in Minimalmedium mit Rohöl reaktiviert.

Obgleich in anderen Habitaten Beimpfungen mit ölabbauenden Organismen bisher weitgehend erfolglos geblieben sind und das lebensfeindliche Meereishabitat eigentlich kein geeigneter Standort für die Bioremediation und Bioaugmentation ist, zeigte sich - wie in den nachfolgenden Beispielen beschrieben - bei Anwendung des Bioremediationsverfahrens nach der Erfindung in mit Öl kontaminierten Meereismesokosmosansätzen und in Gap water (typische flüssige Schicht im antarktischen Sommereis) ein deutlicher Abbau auch langkettiger aliphatischer Öl-Komponenten. Die Ergebnisse der nachfolgend aufgeführten Nachweisbeispiele sind in **Tabelle 2** dargestellt.

### Nachweisbeispiel 1

Es werden Sägespäne, die mit Minimalmedium und mit dem ölabbauenden Meereisbakteriengemisch beladen sind, in Gap water, das sich im antarktischen Meereis ausgebildet hat und welches künstlich mit Rohöl kontaminiert wurde, ausgebracht. Nach einem halben Jahr sind unter Anwendung des Bioremediationsverfahrens nach der Erfindung alle Alkane bis auf einen kleinen Anteil der sehr langkettigen Aliphate komplett abgebaut. In den Vergleichsansätzen nur mit Nährstoffen bzw. ohne Nährstoffzusatz ist ein deutlich geringerer bis gar kein Abbau festzustellen.

### Nachweisbeispiel 2

Im Mesokosmosexperiment wird die Oberfläche von arktischem Meereis (Winterexperiment) bzw. von antarktischem Meereis (Sommer) mit Öl aus der Barentssee mit einem hohen Anteil an Aliphaten kontaminiert. Über die Ölkontamination werden ölabbauende Bakterien, die an Fischmehl immobilisiert sind, verteilt. Es kommt bei diesen Ansätzen zum Abbau von Rohöl unter Anwendung des Bioremediationsverfahrens nach der Erfindung, während im unbehandelten ölkontaminierten Eis kein Abbau festzustellen ist.

Zur Umsetzung des Bioremediationsverfahrens nach der Erfindung können folgende Szenarien beschrieben werden:

### Szenario 1

Kommt es zu einem Ölunfall, in dem größere Mengen an Kohlenwasserstoffen in meereisbedecktes Gebiet gelangen, können große Mengen des Öls, je nach Eisbedingungen, mit Hilfe physikalischer oder mechanischer Methoden entfernt werden. Nach Einsatz dieser Verfahren werden zwar große Ölmengen beseitigt, es werden aber Reste von Kohlenwasserstoffen im Eis verbleiben und es wird ein zerstörtes Ökosystem hinterlassen, sodass kein Abbau der Kohlenwasserstoffe durch die ehemals natürlich im Eis vorkommenden Organismen mehr erfolgen kann. Durch die Ausbringung eines Organismen-Nährstoffgemisches nach der Erfindung kann ein biologischer Abbauprozess schnell in Gang gesetzt werden, sodass auch die verbliebenen Kohlenwasserstoffe in einem akzeptablen Zeitrahmen ganz aus dem Ökosystem entfernt werden können. Das Ausbringen des Bakteriengemisches mit den Nährstoffen auf Trägermaterial kann durch Sprühen aus einer Löschkanone oder vom Helikopter aus auf große Eisflächen sowie Wasserflächen zwischen Eisschollen erfolgen. Hydrophobes Trägermaterial wird sich an die vorhandenen Kohlenwasserstoffe anlagern, wodurch ein direkter Kontakt mit den Mikroorganismen und den Nährstoffen erfolgt. Der weitere Ölabbauprozess erfolgt ohne jedes Dazutun, d.h. die Eisscholle braucht nicht weiter unter Kontrolle gehalten werden. Dieses ist besonders wichtig, da Eisschollen sehr schnell verdriften und auseinander brechen.

### Szenario 2

Kommt es zu einer Kohlenwasserstoff-Kontamination kleineren Ausmaßes, gilt Ähnliches wie oben, nur das dann keine vorherige Reinigung mit Hilfe physikalischer oder mechanischer Methoden erfolgen muss.

### Szenario 3

Kommt es zu einer Leckage beispielsweise in einer Öl-Pipeline, die am Meeresgrund in eisbedeckten Gewässern verläuft (so wie es bereits auf den arktischen Schelfgebieten praktiziert wird, die im Winter und Herbst von Eis bedeckt sind), wird das Öl durch die Wassersäule nach oben steigen und sich unter dem Eis sammeln, wo es schnell in das Eis eingeschlossen wird und so durch Drift der Eisschollen weiträumig verfrachtet werden kann. Das Öl wird im Frühjahr durch Risse und Solekanäle im Eis an die Oberfläche aufsteigen. Durch Ausbringen des Trägermaterials mit den Bakterien und Nährstoffen im Rahmen des Bioremediationsverfahrens nach der Erfindung in die Wassersäule unter dem Eis wird hydrophobes Trägermaterial ebenfalls an der Unterseite des Eises in Kontakt mit dem Öl kommen und in das Eis eingeschlossen werden, sodass der Abbau der Kohlenwasserstoffe schon in der Eissäule beginnen kann. Das oben beschriebene Trägermaterial wird zusammen mit dem Öl an die Eisoberfläche aufsteigen. Der Vorteil dieses Einsatzes ist wiederum, dass das Bakteriengemisch einmal nach dem Unfall ausgebracht wird und dann in situ arbeitet, auch wenn die Eisscholle räumlich verfrachtet wird.

**Tabelle 1**

| **Stamm-Bezeichnung** | **Charakterisierung** | **Verwandtschaft *)** | **Herkunft** |
|---|---|---|---|
| DSMZ-Hinterlegungsnr. Hinterlegungsdatum | | | |
| ***Rhodococcus* GH-1** | breites Abbauspektrum | 98% *R. yunna-nensis* | arktisches Sommer-Eis |
| DSM 18943 | | | |
| DSMZ 22.12.2006 | | | |
| ***Dietzia GH-2*** | breites Abbauspektrum, breites Temperatur- und Salinitätsspektrum | 99,5% *D. maris* | arktisches Winter-Eis |
| DSM 18944 | | | |
| DSMZ 22.12.2006 | | | |
| ***Shewanella GH-4*** | begrenztes Abbauspektrum kurzkettige Alkane, aber sehr schnelles Wachstum bei sehr kalten Temperaturen | 98% S. livingstonensis | arktisches Sommer-Eis |
| DSM 18946 | | | |
| DSMZ 22.12.2006 | | | |
| ***Marinobacter GH-9*** | gutes Abbauspektrum, sehr schnelles Wachstum | 95% *M. aquaeolei* VT8 | antarktisches Sommer-Eis |
| DSM 18951 | | | |
| DSMZ 22.12.2006 | | | |
| ***Pseudomonas GH-10*** | breites Abbauspektrum, besonders gut im Zusammenspiel | 94% *P. pertucinogena* | arktisches Sommer-Eis |
| DSM 18952 | | | |
| DSMZ 22.12.2006 | | | |
| ***Oleispira GH-11*** | breites Alkan-Abbauspektrum bei sehr niedriger Temperatur, Stamm empfindlich | 99,6% *O.antarctica* | arktisches Winter-Eis |
| DSM 18953 | | | |
| DSMZ 22.12.2006 | | | |
| Zusätzliche Stämme | | | |
| ***Marinobacter GH-3*** | sehr breites Abbauspektrum, psychrotolerant | 96% *M*. *aquaeolei* VT8 | arktisches Winter-Eis |
| DSM 18945 | | | |
| DSMZ 22.12.2006 | | | |
| ***Marinomonas GH-5*** | Abbau spezifischer Kohlenwasserstoffe , schnelles Wachstum bei Minustemperaturen | 98% *M. protea* | Gemischtes Eis |
| DSM 18947 | | | |
| DSMZ 22.12.2006 | | | |
| ***Pseudoalteromonas GH-6*** | Abbau spezifischer Kohlenwasserstoffe sehr breites Temperaturspektrum | 99,6% *P. elyakovii* | arktisches Sommer-Eis |
| DSM 18948 | | | |
| DSMZ 22.12.2006 | | | |
| ***Psychrobacter GH-7*** | Abbau spezifischer Kohlenwasserstoffe, sehr breites Salinitätsspektrum | 98% *P. glacincola* | arktisches Sommer-Eis |
| DSM 18949 | | | |
| DSMZ 22.12.2006 | | | |
| ***Jannaschia GH-8*** | breites Abbauspektrum, langsames Wachstum | 94% *J. rubra* | antarktisches Sommer-Eis |
| DSM 18950 | | | |
| DSMZ 22.12.2006 | | | |

| | | | |
|---|---|---|---|
| *) Sequenzähnlichkeit des 16S rRNA Gens mit dem am nächsten verwandten Typstamm oder gut beschriebenen Stamm | | | |

**Tabelle 2**

| | nach einem halben Jahr mit Bioaugmentation plus Nährstoffzusatz | | nach einem halben Jahr nur mit Nährstoffzusatz | | nach einem halben Jahr ohne Zusätze | |
|---|---|---|---|---|---|---|
| Probe | n-Alkane | Pristane, Phytane | n-Alkane | Pristane, Phytane | n-Alkane | Pristane Phytane |
| Gap water von antarktischem Meereis | C₁₄-C₁₅ (g)^{#)}, | pr ph (l) | C₁₄-C₁₅ (g) | pr ph (k) | C₁₄-C₁₅ (l) | pr ph (k) |
| | C₁₆-C₁₉ (g) | | C₁₆-C₁₉ (t) | | C₁₆-C₁₉ (k) | |
| | C₂₀-C₂₄ (g) | | C₂₀-C₂₄ (k) | | C₂₀-C₂₄ (k) | |
| | C₂₄-C₃₀ (t) | | C₂₄-C₃₀ (k | | C₂₄-C₃₀ (k) | |
| Mesokosmosexperiment mit arktischem Meereis | C₁₄-C₁₅ (g) | pr ph (l) | C₁₄-C₁₅ (g), | pr ph (k) | C₁₄-C₁₅ (l) | pr ph (k) |
| | C₁₆-C₁₉ (g) | | C₁₆-C₁₉ (t) | | C₁₆-C₁₉ (k) | |
| | C₂₀-C₂₄ (g) | | C₂₀-C₂₄ (k) | | C₂₀-C₂₄ (k) | |
| | C₂₄-C₃₀ (t) | | C₂₄-C₃₀ (k) | | C₂₄-C₃₀ (k) | |

| | | | | | | |
|---|---|---|---|---|---|---|
| #) Abbaustärke: ganz (g), teilweise (t), leicht (I), kein sichtbarer Abbau (k) | | | | | | |

## Patentansprüche

1. Bioremediationsverfahren zum beschleunigten biologischen Abbau von Petroleum-Kohlenwasserstoffen in den polaren meereisbedeckten Regionen durch Bioaugmentation mit exogenen kohlenwasserstoffabbauenden Bakterien und durch Zugabe von Nährstoffen,
gekennzeichnet durch
eine Inkontaktbringung der abzubauenden Petroleum-Kohlenwasserstoffe mit einem Inokulum aus zumindest nachfolgenden Komponenten:
• einem Bakteriengemisch aus unterschiedlichen kälteangepassten, autochthonen Bakterienstämmen, die gewinnbar sind durch
• Ansetzen eines labortechnischen Mesokosmos mit Meereis von einem aktuellen oder potenziellen Abbauort,
• künstliche Kontamination des Mesokosmos mit Rohöl,
• Versehen des Mesokosmos mit Nährstoffen,
• Inkubation des Mesokosmos bei -3°C über einen Zeitraum von 12 bis 36 Monaten in der Form, dass die Eis-Wassersituation stabil bleibt,
• Isolation der dominanten Bakterienstämme und
• Auswahl der isolierten Bakterienstämme danach, dass sie
• auch bei einer Umgebungstemperatur -3°C aktiv sind,
• einen unterschiedlichen Temperaturtoleranzbereich aufweisen,
• einen unterschiedlichen Salztoleranzbereich besitzen,
• ein unterschiedliches Abbauspektrum haben und
• ein unterschiedliches Potenzial aufweisen Öl zu emulgieren,
• den Nährstoffen und
• einem umweltfreundlichen Trägermaterial, an dem zumindest die Bakterien der Bakterienstämme immobilisiert sind.

2. Bioremediationsverfahren nach Anspruch 1,
gekennzeichnet durch
einen Aufbau des Bakteriengemischs zumindest aus den nachfolgend ausgeführten Bakterienstämmen für einen Einsatz in arktischen meereisbedeckten Regionen:
• *Rhodococcus* GH-1 (DSM 18943, DSMZ 22.12.2006)
• *Dietzia* GH-2 (DSM 18944, DSMZ 22.12.2006)
• *Shewanella* GH-4 (DSM 18946, DSMZ 22.12.2006)
• *Marinobacter* GH-9 (DSM 18951, DSMZ 22.12.2006)
• *Pseudomonas* GH-10 (DSM 18952, DSMZ 22.12.2006)
• *Oleispira* GH-11 (DSM 18953, DSMZ 22.12.2006).

3. Bioremediationsverfahren nach Anspruch 1 oder 2,
gekennzeichnet durch
eine Zufügung von zumindest einem weiteren Bakterienstamm zum Bakteriengemisch zur Erweiterung des Abbauspektrums und der Fähigkeit zum Emulgieren des Öls.

4. Bioremediationsverfahren nach Anspruch 3,
gekennzeichnet durch
eine Zufügung von zumindest einem der nachfolgend ausgeführten Bakterienstämme zum Bakteriengemisch:
• *Marinobacter* GH-3 (DSM 18945, DSMZ 22.12.2006)
• *Marinomonas* GH-5 (DSM 18947, DSMZ 22.12.2006)
• *Pseudoalteromonas* GH-6 (DSM 18948, DSMZ 22.12.2006)
• *Psychrobacter GH-7* (DSM 18949, DSMZ 22.12.2006)
• *Jannaschia* GH-8 (DSM 18950, DSMZ 22.12.2006)

5. Bioremediationsverfahren nach einem der Ansprüche 1 bis 4, gekennzeichnet durch
eine genetische Veränderung der eingesetzten Bakterienstämme.

6. Bioremediationsverfahren nach einem der Ansprüche 1 bis 5, gekennzeichnet durch
einen Einsatz der aus den Bakterienstämmen erhältlichen Enzymzusammensetzungen zusätzlich zu den oder anstelle der eingesetzten Bakterienstämme, wobei die Enzymzusammensetzungen mit Hilfe von Trägermaterial immobilisiert sind.

7. Bioremediationsverfahren nach einem der Ansprüche 1 bis 6, gekennzeichnet durch
eine mehrfache Inkontaktbringung des Inokulums mit den abzubauenden Petroleum-Kohlenwasserstoffen mit einer unterschiedlichen Zusammensetzung des Inokulums hinsichtlich der eingesetzten Bakterienstämme und/oder der eingesetzten Nährstoffe und/oder der eingesetzten Trägermaterialien.

8. Bioremediationsverfahren nach einem der Ansprüche 1 bis 7, gekennzeichnet durch
einen gleichen Volumenanteil aller eingesetzten Bakterienstämme und/oder deren Enzymzusammensetzungen im Inokulum.

9. Bioremediationsverfahren nach einem der Ansprüche 1 bis 8, gekennzeichnet durch
eine Inkontaktbringung der abzubauenden Petroleum-Kohlenwasserstoffe mit dem Inokulum bei einer Umgebungstemperatur unterhalb von +7°C.

10. Bioremediationsverfahren nach einem der Ansprüche 1 bis 9, gekennzeichnet durch
organische oder anorganische Nährstoffe mit einem hohen Anteil von Stickstoff und Phosphat.

11. Bioremediationsverfahren nach einem der Ansprüche 1 bis 10, gekennzeichnet durch
eine Immobilisierung auch der Nährstoffe an dem Trägermaterial.

12. Bioremediationsverfahren nach einem der Ansprüche 1 bis 11, gekennzeichnet durch
eine Inkonfaktbringung der abzubauenden Petroleum-Kohlenwasserstoffe mit dem Inokulum in fester Partikelform durch Ausstreuen auf dem Meereis und/oder auf der Wasseroberfläche.

13. Bioremediationsverfahren nach einem der Ansprüche 1 bis 11, gekennzeichnet durch
eine Inkontaktbringung der abzubauenden Petroleum-Kohlenwasserstoffe mit dem Inokulum in flüssiger Form durch
• Ausgießen oder Versprühen auf das Meereis und/oder
• durch Einleiten oder Pumpen in die Wassersäule unter das Meereis.

14. Bioremediationsverfahren nach Anspruch 12 oder 13,
gekennzeichnet durch
Ausbringen des Inokulums mit einer Löschkanone von einem Schiff oder einem Flugzeug oder Helikopter aus.

15. Bioremediationsverfahren nach einem der Ansprüche 1 bis 14, gekennzeichnet durch
den Einsatz von zumindest zwei verschiedenen Trägermaterialien.

16. Bioremediationsverfahren nach einem der Ansprüche 1 bis 15, gekennzeichnet durch
den Einsatz von hydrophoben und/oder hydrophilen Trägermaterial in Abhängigkeit vom Auftreten der abzubauenden Petroleum-Kohlenwasserstoffe auf oder im Meereis und/oder auf oder im Meerwasser.

17. Bioremediationsverfahren nach einem der Ansprüche 1 bis 16, gekennzeichnet durch
unterschiedlich aufgebaute Trägermaterialien in Partikelform in Form von
• anorganischen Nährstoffen, umgeben von einer Fettsäurehülle,
• Kugeln aus Aramid-Polymer und Aktivkohle mit einer Membranumhüllung,
• faserbildenden Proteinen,
• Fischmehl umhüllt mit einem Polyoxyethylen(20)-sorbitanmonolaurat oder
• Sägespänen.

18. Bioremediationsverfahren nach einem der Ansprüche 1 bis 17, gekennzeichnet durch
eine Ausbildung des Trägermaterials gleichzeitig als Nährstoffquelle.

19. Bioremediationsverfahren nach einem der Ansprüche 1 bis 18, gekennzeichnet durch
eine Lagerung der Bakterienstämme in frischem oder konserviertem Zustand bis zum Einsatzfall, wobei die Lagerung in frischem Zustand in einer Nährlösung, angereichert mit Kohlenwasserstoffen, erfolgt.

20. Bioremediationsverfahren nach Anspruch 19,
gekennzeichnet durch
eine Anzucht der frischen oder wiederbelebten Bakterienstämme bei Temperaturen unterhalb +7°C in einem Mineralmedium mit Zusatz von Petroleum-Kohlenwasserstoffen bis zu einer Bakteriendichte von mindestens 1x10⁹ Bakterien pro ml Flüssigkeit.

21. Bioremediationsverfahren nach einem der Ansprüche 1 bis 20 gekennzeichnet durch
ein Zusatzverfahren im Nachgang zum aeroben Abbau von großen Mengen von Petroleum-Kohlenwasserstoffen.

22. Bioremediationsverfahren nach einem der Ansprüche 1 bis 21 gekennzeichnet durch
einen Einsatz auch zur Wiederherstellung der natürlichen Mikroflora am Einsatzort.

23. Bakteriengemisch als Mittel zur Durchführung des Bioremediationsverfahrens zum beschleunigten biologischen Abbau von Petroleum-Kohlenwasserstoffen in den polaren meereisbedeckten Regionen durch Bioaugmentation mit exogenen kohlenwasserstoffabbauenden Bakterien und durch Zugabe von Nährstoffen nach einem der Ansprüche 1 bis 22, gekennzeichnet durch
eine Kombination von Bakterienstämmen, die gewinnbar sind durch eine Anreicherung in einem labortechnischen Mesokosmosaufbau über einen Zeitraum von 12 bis 36 Monaten, anschließende Isolation der dominanten Bakterienstämme und Auswahl der isolierten Bakterienstämme danach, dass sie auch bei einer Umgebungstemperatur -3°C aktiv sind, einen unterschiedlichen Temperaturtoleranzbereich aufweisen, einen unterschiedlichen Salztoleranzbereich besitzen, ein unterschiedliches Abbauspektrum haben und ein unterschiedliches Potenzial aufweisen Öl zu emulgieren, wobei der Mesokosmos mit Meereis von einem aktuellen oder potenziellen Abbauort angesetzt, künstlich mit Rohöl kontaminiert, mit Nährstoffen versehen und bei - 3°C so inkubiert wird, dass die Eis-Wassersituation stabil bleibt.

24. Bakteriengemisch nach Anspruch 23,
gekennzeichnet durch
zumindest den nachfolgend ausgeführten Bakterienstämmen, die bei der DSMZ - Deutsche Sammlung von Mikroorganismen und Zellkulturen GmbH, Braunschweig, Deutschland - hinterlegt sind und folgende Bezeichnung und Eingangsnummer haben:
• *Rhodococcus* GH-1 (DSM 18943, DSMZ 22.12.2006)
• *Dietzia* GH-2 (DSM 18944, DSMZ 22.12.2006)
• *Shewanella* GH-4 (DSM 18946, DSMZ 22.12.2006)
• *Marinobacter* GH-9 (DSM 18951, DSMZ 22.12.2006)
• *Pseudomonas* GH-10 (DSM 18952, DSMZ 22.12.2006)
• *Oleispira* GH-11 (DSM 18953, DSMZ 22.12.2006).

25. Bakteriengemisch nach Anspruch 23 oder 24,
gekennzeichnet durch
eine Zufügung zum Bakteriengemisch von zumindest einem der nachfolgend ausgeführten Bakterienstämme zur Erweiterung des Abbauspektrums und der Fähigkeit zur Emulgierung von Öl, die bei der DSMZ - Deutsche Sammlung von Mikroorganismen und Zellkulturen GmbH, Braunschweig, Deutschland - hinterlegt sind und folgende Bezeichnung und Eingangsnummer haben:
• *Marinobacter GH-3* (DSM 18945, DSMZ 22.12.2006)
• *Marinomonas* GH-5 (DSM 18947, DSMZ 22.12.2006)
• *Pseudoalteromonas* GH-6 (DSM 18948, DSMZ 22.12.2006)
• *Psychrobacter*GH-7 (DSM 18949, DSMZ 22.12.2006)
• *Jannaschia* GH-8 (DSM 18950, DSMZ 22.12.2006)

26. Bakteriengemisch nach einem der Ansprüche 23 bis 25, gekennzeichnet durch
eine genetische Veränderung der Bakterienstämme.

27. Bakteriengemisch nach einem der Ansprüche 23 bis 26, gekennzeichnet durch
eine Hinterlegung der einzelnen bekannten Bakterienstämme in einem Archiv zum schnellen Zugriff auf die einzelnen Bakterienstämme in Abhängigkeit vom speziellen Einsatzfall des Bioremediationsverfahrens.

28. Enzymgemisch zum Abbau von Kohlenwasserstoffen als Mittel zur Durchführung des Bioremediationsverfahrens zum beschleunigten biologischen Abbau von Petroleum-Kohlenwasserstoffen in den polaren meereisbedeckten Regionen durch Bioaugmentation mit exogenen kohlenwasserstoffabbauenden Bakterien unter Zugabe von Nährstoffen nach einem der Ansprüche 6 bis 13, 15 bis 19 und 21 bis 22,
gekennzeichnet durch
eine Kombination unterschiedlicher Enzyme oder Enzymzusammensetzungen, die gewinnbar sind aus den Bakterienstämmen zur Gewinnung der Bakteriengemische nach einem der Ansprüche 23 bis 27, wobei die Bakterienstämme danach ausgewählt sind, dass sie auch bei einer Umgebungstemperatur -3°C aktiv sind, einen unterschiedlichen Temperaturtoleranzbereich aufweisen, einen unterschiedlichen Salztoleranzbereich besitzen, ein unterschiedliches Abbauspektrum haben und ein unterschiedliches Potenzial aufweisen Öl zu emulgieren.

## Claims

1. A bioremediation method for accelerated biological degradation of petroleum hydrocarbons in sea ice - covered polar regions by bioaugmentation with exogenous hydrocarbon degrading bacteria and with the addition of nutrients,
**characterized by**
bringing the petroleum hydrocarbons to be degraded into contact with an inoculum formed from at least the following components:
• a mixture of bacteria formed from different cold-adapted autochthonous bacterial strains which can be obtained by
• setting up a laboratory mesocosm with sea ice from a current or potential degradation site,
• artificially contaminating the mesocosm with crude oil,
• providing the mesocosm with nutrients,
• incubating the mesocosm at -3°C for a period of 12 to 36 months in a form in which the ice/water situation remains stable,
• isolating the dominant bacterial strains, and
• selecting the isolated bacterial strains in a manner such that they:
• are also active at an ambient temperature of -3°C,
• have a different temperature tolerance range,
• have a different salt tolerance range,
• have a different degradation spectrum, and
• have a different potential for emulsifying oil,
• the nutrients, and
• an environmentally friendly support material on which at least the bacteria of the bacterial strain are immobilized.

2. The bioremediation method as claimed in claim 1, **characterized by**
the bacterial mixture being formed from at least the following bacterial strains for use in arctic sea ice-covered regions:
• *Rhodococcus* GH-1 (DSM 18943, DSMZ 22.12.2006)
• *Dietzia* GH-2 (DSM 18944, DSMZ 22.12.2006)
• *Shewanella* GH-4 (DSM 18946, DSMZ 22.12.2006)
• *Marinobacter* GH-9 (DSM 18951, DSMZ 22.12.2006)
• *Pseudomonas* GH-10(DSM 18952, DSMZ 22.12.2006)
• *Oleispira* GH-11(DSM 18943, DSMZ 22.12.2006).

3. The bioremediation method as claimed in claim 1 or claim 2,
**characterized by**
adding at least one further bacterial strain to the bacterial mixture to broaden the degradation spectrum and the oil emulsification capacity.

4. The bioremediation method as claimed in claim 3, **characterized by**
adding at least one of the following bacterial strains to the bacterial mixture:
• *Marinobacter* GH-3(DSM 18945, DSMZ 22.12.2006)
• *Marinomonas* GH-5(DSM 18947, DSMZ 22.12.2006)
• *Pseudoalteromonas* GH-6 (DSM 18948, DSMZ 22.12.2006)
• *Psychrobacter* GH-7(DSM 18949, DSMZ 22.12.2006)
• *Jannaschia* GH-8(DSM 18950, DSMZ 22.12.2006).

5. The bioremediation method as claimed in one of claims 1 to 4,
**characterized by**
a genetic change in the bacterial strains employed.

6. The bioremediation method as claimed in one of claims 1 to 5,
**characterized by**
using the enzyme compositions obtainable from the bacterial strains in addition to or instead of the bacterial strains employed, wherein the enzyme compositions are immobilized with the aid of support material.

7. The bioremediation method as claimed in one of claims 1 to 6,
**characterized by**
bringing the inoculum into contact with the petroleum hydrocarbons to be degraded a plurality of times, with a different composition of the inoculum as regards the bacterial strains employed and/or the nutrients employed and/or the support material employed.

8. The bioremediation method as claimed in one of claims 1 to 7,
**characterized by**
the same volume fraction of all bacterial strains employed and/or their enzyme compositions in the inoculum.

9. The bioremediation method as claimed in one of claims 1 to 8,
**characterized by**
bringing the petroleum hydrocarbons to be degraded into contact with the inoculum at an ambient temperature of below +7°C.

10. The bioremediation method as claimed in one of claims 1 to 9,
**characterized by**
organic or inorganic nutrients with a high level of nitrogen and phosphate.

11. The bioremediation method as claimed in one of claims 1 to 10,
**characterized by**
immobilizing the nutrients on the support material as well.

12. The bioremediation method as claimed in one of claims 1 to 11,
**characterized by**
bringing the petroleum hydrocarbons to be degraded into contact with the inoculum in the form of solid particles by scattering onto the sea ice and/or onto the surface of the water.

13. The bioremediation method as claimed in one of claims 1 to 11,
**characterized by**
bringing the petroleum hydrocarbons to be degraded into contact with the inoculum in liquid form by:
• pouring or spraying onto the sea ice and/or
• by introducing or pumping into the column of water under the sea ice.

14. The bioremediation method as claimed in claim 12 or 13,
**characterized by**
deploying the inoculum from a ship or an aircraft or helicopter by means of a water cannon.

15. The bioremediation method as claimed in one of claims 1 to 14,
**characterized by**
using at least two different support materials.

16. The bioremediation method as claimed in one of claims 1 to 15,
**characterized by**
using hydrophobic and/or hydrophilic support materials depending on whether the petroleum hydrocarbons to be degraded are deployed on or in sea ice and/or on or in sea water.

17. The bioremediation method as claimed in one of claims 1 to 16,
**characterized by**
differently constructed support means in the form of particles in the form of
• inorganic nutrients surrounded by a fatty acid covering,
• beads formed from aramid polymer and activated charcoal with a membrane covering,
• fibre-forming proteins,
• fish meal covered with a polyoxyethylene(20) sorbitan monolaurate, or
• sawdust.

18. The bioremediation method as claimed in one of claims 1 to 17,
**characterized by**
simultaneously configuring the support material as a nutrient source.

19. The bioremediation method as claimed in one of claims 1 to 18,
**characterized by**
storing the bacterial strains in the fresh or preserved state until deployment, wherein storage in the fresh state is carried out in a nutrient solution enriched with hydrocarbons.

20. The bioremediation method as claimed in claim 19, **characterized by**
culturing the fresh or resuscitated bacterial strains at temperatures below +7°C in a mineral medium with the addition of petroleum hydrocarbons until the bacterial density is at least 1 × 10⁹ bacteria per mL of liquid.

21. The bioremediation method as claimed in one of claims 1 to 20,
**characterized by**
a subsequent additional method following aerobic degradation of large quantities of petroleum hydrocarbons.

22. The bioremediation method as claimed in one of claims 1 to 21,
**characterized by**
an additional application for restoring the natural microflora at the point of application.

23. A bacterial mixture as a means for carrying out a bioremediation method for accelerated biological degradation of petroleum hydrocarbons in sea ice covered polar regions by bioaugmentation with exogenous hydrocarbon degrading bacteria and with the addition of nutrients as claimed in one of claims 1 to 22,
**characterized by**
a combination of bacterial strains which can be obtained by enrichment in a laboratory mesocosm set-up over a period of 12 to 36 months, subsequent isolation of the dominant bacterial strains and selection of the isolated bacterial strains in a manner such that they are also active at an ambient temperature of -3°C, they exhibit a different temperature tolerance range, they have a different salt tolerance range, they have a different degradation spectrum and they exhibit a different potential for emulsifying oil, wherein the mesocosm is set up with sea ice from a current or potential degradation location, is artificially contaminated with crude oil, is provided with nutrients and is incubated at -3°C such that the ice/water situation remains stable.

24. The bacterial mixture as claimed in claim 23, **characterized by**
at least the following bacterial strains which have been deposited with the DSMZ - Deutsche Sammlung von Microorganismen und Zellkulturen GmbH - (German Collection of Microorganisms and Cell Cultures) in Braunschweig, Germany and have been assigned the following designation and accession numbers:
• *Rhodococcus* GH-1 (DSM 18943, DSMZ 22.12.2006)
• *Dietzia* GH-2 (DSM 18944, DSMZ 22.12.2006)
• *Shewanella* GH-4 (DSM 18946, DSMZ 22.12.2006)
• *Marinobacter* GH-9 (DSM 18951, DSMZ 22.12.2006)
• *Pseudomonas* GH-10(DSM 18952, DSMZ 22.12.2006)
• *Oleispira* GH-11(DSM 18943, DSMZ 22.12.2006).

25. The bacterial mixture as claimed in claim 23 or 24,
**characterized by**
adding to the bacterial mixture at least one of the following bacterial strains in order to broaden the degradation spectrum and the oil emulsification ability, which have been deposited with the DSMZ - Deutsche Sammlung von Microorganismen und Zellkulturen GmbH - (German Collection of Microorganisms and Cell Cultures) in Braunschweig, Germany and have been assigned the following designation and accession numbers:
• *Marinobacter* GH-3(DSM 18945, DSMZ 22.12.2006)
• *Marinomonas* GH-5(DSM 18947, DSMZ 22.12.2006)
• *Pseudoalteromonas* GH-6 (DSM 18948, DSMZ 22.12.2006)
• *Psychrobacter* GH-7(DSM 18949, DSMZ 22.12.2006)
• *Jannaschia* GH-8(DSM 18950, DSMZ 22.12.2006).

26. The bacterial mixture as claimed in one of claims 23 to 25,
**characterized by**
a genetic change in the bacterial strain.

27. The bacterial mixture as claimed in one of claims 23 to 26,
**characterized by**
depositing the individually known bacterial strains in an archive in order to obtain rapid access to the individual bacterial strains as a function of the mode of deployment of the application of the bioremediation method.

28. A mixture of enzymes for the degradation of hydrocarbons as a means for carrying out the bioremediation method for accelerated biological degradation of petroleum hydrocarbons in the polar sea ice-covered regions by bioaugmentation with exogenous hydrocarbon degrading bacteria by adding nutrients as claimed in one of claims 6 to 13, 15 to 19 and 21 to 22,
**characterized by**
a combination of different enzymes or enzyme compositions which can be obtained from the bacterial strains by obtaining the bacterial mixtures as claimed in one of claims 23 to 27,
wherein the bacterial strains are selected such that they are also active at an ambient temperature of -3°C, exhibit a different temperature tolerance range, have a different salt tolerance range, have a different degradation spectrum and exhibit a different potential for oil emulsification.

## Revendications

1. Procédé de bioremédiation permettant une biodégradation accélérée d'hydrocarbures d'huile minérale dans des régions polaires couvertes par une banquise, en réalisant une bioaugmentation avec des bactéries exogènes capables de dégrader des hydrocarbures et en ajoutant des éléments nutritifs,
**caractérisé par**
une mise en contact des hydrocarbures d'huile minérale à dégrader avec un inoculum ayant au moins les constituants suivants :
• un mélange de bactéries réalisé à partir de différentes souches de bactéries autochtones psychrotolérantes pouvant être obtenues par
• la préparation d'un mésocosme à l'échelle laboratoire, contenant de la glace d'eau de mer issue d'un site de dégradation réel ou potentiel,
• la contamination artificielle dudit mésocosme avec du pétrole brut,
• l'ajout d'éléments nutritifs audit mésocosme,
• l'incubation dudit mésocosme à -3°C pendant une durée comprise entre 12 et 36 mois de sorte que le rapport glace/eau reste stable,
• l'isolation des souches de bactéries dominantes, et
• la sélection de souches de bactéries isolées telle que celles-ci
• maintiennent leur activité à une température ambiante de -3 °C,
• sont tolérantes vis-à-vis de différentes plages de température,
• sont tolérantes vis-à-vis de différentes plages de salinité,
• se distinguent les unes des autres en ce qui concerne leur capacité à dégrader des matières, et
• se distinguent les unes des autres en ce qui concerne leur capacité à émulsifier l'huile,
• les éléments nutritifs, et
• au moins un matériau support écologique sur lequel sont immobilisées au moins les bactéries desdites souches de bactéries.

2. Procédé de bioremédiation selon la revendication 1,
**caractérisé par**
une constitution dudit mélange de bactéries au moins à partir des souches bactéries détaillées ci-après, pour une mise en oeuvre dans des régions arctiques couvertes par une banquise :
• *Rhodococcus* GH-1 (DSM 18943, DSMZ 22/12/2006)
• *Dietzia* GH-2 (DSM 18944, DSMZ 22/12/2006)
• *Shewanella* GH-4 (DSM 18946, DSMZ 22/12/2006)
• *Marinobacter* GH-9 (DSM 18951, DSMZ 22/12/2006)
• *Pseudomonas* GH-10 (DSM 18952, DSMZ 22/12/2006)
• *Oleispira* GH-11 (DSM 18953, DSMZ 22/12/2006).

3. Procédé de bioremédiation selon les revendications 1 ou 2,
**caractérisé par**
un ajout d'au moins une autre souche de bactéries audit mélange de bactéries, pour ainsi élargir les capacités à dégrader des matières et les capacités à émulsifier ladite huile.

4. Procédé de bioremédiation selon la revendication 3,
**caractérisé par**
un ajout audit mélange de bactéries d'au moins une des souches de bactéries détaillées ci-après :
• *Marinobacter* GH-3 (DSM 18945, DSMZ 22/12/2006)
• *Marinomonas* GH-5 (DSM 18947, DSMZ 22/12/2006)
• *Pseudoalteromonas* GH-6 (DSM 18948, DSMZ 22/12/2006)
• *Psychrobacter* GH-7 (DSM 18949, DSMZ 22/12/2006)
• *Jannaschia* GH-8 (DSM 18950, DSMZ 22/12/2006)

5. Procédé de bioremédiation selon l'une des revendications 1 à 4,
**caractérisé par**
une modification génétique des souches de bactéries mises en oeuvre.

6. Procédé de bioremédiation selon l'une des revendications 1 à 5,
**caractérisé par**
une mise en oeuvre de compositions d'enzymes pouvant être obtenues à partir desdites souches de bactéries, de manière supplémentaire aux souches de bactéries mises en oeuvre ou à la place de ces dernières, les compositions d'enzymes étant immobilisées à l'aide d'un matériau support.

7. Procédé de bioremédiation selon l'une des revendications 1 à 6,
**caractérisé par**
une mise en contact, à plusieurs reprises, dudit inoculum avec les hydrocarbures d'huile minérale à dégrader, en faisant varier la composition de l'inoculum en ce qui concerne les souches de bactéries mises en oeuvre et/ou les éléments nutritifs mis en oeuvre et/ou les matériaux support mis en oeuvre.

8. Procédé de bioremédiation selon l'une des revendications 1 à 7,
**caractérisé par**
une proportion volumique égale de toutes des souches de bactéries et/ou leurs compositions d'enzymes mises en oeuvre dans ledit inoculum.

9. Procédé de bioremédiation selon l'une des revendications 1 à 8,
**caractérisé par**
une mise en contact des hydrocarbures d'huile minérale à dégrader avec ledit inoculum à une température ambiante inférieure à +7 °C.

10. Procédé de bioremédiation selon l'une des revendications 1 à 9,
**caractérisé par**
des éléments nutritifs organiques ou inorganiques ayant une proportion élevée en azote et phosphate.

11. Procédé de bioremédiation selon l'une des revendications 1 à 10,
**caractérisé en ce que**
lesdits éléments nutritifs sont également immobilisés sur ledit matériau de support.

12. Procédé de bioremédiation selon l'une des revendications 1 à 11,
**caractérisé par**
une mise en contact des hydrocarbures d'huile minérale à dégrader avec ledit inoculum sous sa forme solide particulaire, en répandant ce dernier sur la banquise et/ou sur la surface de l'eau.

13. Procédé de bioremédiation selon l'une des revendications 1 à 11,
**caractérisé par**
une mise en contact des hydrocarbures d'huile minérale à dégrader avec ledit inoculum sous sa forme liquide,
• en versant ou nébulisant ce dernier sur la banquise et/ou
• en injectant ou pompant ce dernier dans la colonne d'eau sous la banquise.

14. Procédé de bioremédiation selon les revendications 12 ou 13,
**caractérisé en ce que**
ledit inoculum est répandu à l'aide d'un canon à eau depuis un navire ou un avion ou hélicoptère.

15. Procédé de bioremédiation selon l'une des revendications 1 à 14,
**caractérisé par**
une mise en oeuvre d'au moins deux matériaux support différents.

16. Procédé de bioremédiation selon l'une des revendications 1 à 15,
**caractérisé par**
la mise en oeuvre de matériaux support hydrophobes et/ou hydrophiles en fonction d'une présence des hydrocarbures d'huile minérale à dégrader sur ou dans la banquise et/ou sur ou dans l'eau de mer.

17. Procédé de bioremédiation selon l'une des revendications 1 à 16,
**caractérisé par**
des matériau support particulaires présentant différentes structures et ayant la forme
• d'éléments nutritifs inorganiques, entourés d'une enveloppe en acides gras,
• de sphères en polymère d'aramide et en charbon actif, pourvues d'une enveloppe membranaire,
• de protéines formant des fibres,
• de farine de poisson, enveloppé par du monolaurate de polyoxyéthylène(20)-sorbitane,
ou
• de sciure de bois.

18. Procédé de bioremédiation selon l'une des revendications 1 à 17,
**caractérisé par**
une réalisation du matériau support de manière ce que celui-ci serve également de source d'éléments nutritifs.

19. Procédé de bioremédiation selon l'une des revendications 1 à 18,
**caractérisé par**
un stockage desdites souches de bactéries, à l'état frais ou conservé, jusqu'à leur mise en oeuvre, le stockage à l'état frais ayant lieu dans un bouillon de culture enrichi en hydrocarbures.

20. Procédé de bioremédiation selon la revendication 19,
**caractérisé par**
une culture des souches de bactéries fraîches ou reconstituées à des températures inférieures à +7 °C dans un milieu à base de minéraux ayant subi en ajout d'hydrocarbures d'huile minérale, jusqu'à atteindre une densité de bactéries d'au moins 1 x 10⁹ bactéries par ml de liquide.

21. Procédé de bioremédiation selon l'une des revendications 1 à 20,
**caractérisé par**
un procédé d'appoint, suite à la dégradation aérobie de grandes quantités d'hydrocarbures d'huile minérale.

22. Procédé de bioremédiation selon l'une des revendications 1 à 21,
**caractérisé par**
une mise en oeuvre visant également à rétablir la microflore naturelle sur le site de mise en oeuvre.

23. Mélange de bactéries permettant de mettre en oeuvre le procédé de bioremédiation qui permet une biodégradation accélérée d'hydrocarbures d'huile minérale dans des régions polaires couvertes par une banquise, en réalisant une bioaugmentation avec des bactéries exogènes capables de dégrader des hydrocarbures et en ajoutant des éléments nutritifs, selon l'une des revendications 1 à 22, **caractérisé par**
une association de souches de bactéries pouvant être obtenues par un enrichissement au sein d'une structure de mésocosme à l'échelle laboratoire pendant une période comprise entre 12 et 36 mois,
suivi d'une isolation des souches de bactéries dominantes et d'une sélection de souches de bactéries isolées telle que celles-ci maintiennent leur activité à une température ambiante de -3 °C, sont tolérantes vis-à-vis de différentes plages de température, sont tolérantes vis-à-vis de différentes plages de salinité, se distinguent les unes des autres en ce qui concerne leur capacité à dégrader des matières, et se distinguent les unes des autres en ce qui concerne leur capacité à émulsifier l'huile, ledit mésocosme étant préparé de manière à ce qu'il contienne de la glace d'eau de mer issue d'un site de dégradation réel ou potentiel, contaminé artificiellement avec du pétrole brut, soumis à l'ajout d'éléments nutritifs et soumis à une incubation à -3 °C de sorte que le rapport glace/eau reste stable.

24. Mélange de bactéries selon la revendication 23, **caractérisé par**
au moins les souches de bactéries détaillées ci-après, lesquelles ont été déposées auprès de la DSMZ - Deutsche Sammlung von Mikroorganismen und Zellkulturen GmbH, Braunschweig, Allemagne - et portent les désignations et numéros d'entrée suivants :
• *Rhodococcus* GH-1 (DSM 18943, DSMZ 22/12/2006)
• *Dietzia* GH-2 (DSM 18944, DSMZ 22/12/2006)
• *Shewanella* GH-4 (DSM 18946, DSMZ 22/12/2006)
• *Marinobacter* GH-9 (DSM 18951, DSMZ 22/12/2006)
• *Pseudomonas* GH-10 (DSM 18952, DSMZ 22/12/2006)
• *Oleispira* GH-11 (DSM 18953, DSMZ 22/12/2006).

25. Mélange de bactéries selon les revendications 23 ou 24,
**caractérisé par**
un ajout audit mélange de bactéries d'au moins une des souches de bactéries détaillées ci-après, lesquelles permettent d'élargir les capacités à dégrader des matières et les capacités à émulsifier de l'huile, et lesquelles ont été déposées auprès de la DSMZ - Deutsche Sammlung von Mikroorganismen und Zellkulturen GmbH, Braunschweig, Allemagne - et portent les désignations et numéros d'entrée suivants :
• *Marinobacter* GH-3 (DSM 18945, DSMZ 22/12/2006)
• *Marinomonas* GH-5 (DSM 18947, DSMZ 22/12/2006)
• *Pseudoalteromonas* GH-6 (DSM 18948, DSMZ 22/12/2006)
• *Psychrobacter* GH-7 (DSM 18949, DSMZ 22/12/2006)
• *Jannaschia* GH-8 (DSM 18950, DSMZ 22/12/2006)

26. Mélange de bactéries selon l'une des revendications 23 à 25,
**caractérisé par**
une modification génétique desdites souches de bactéries.

27. Mélange de bactéries selon l'une des revendications 23 à 26,
**caractérisé par**
une conservation des différentes souches de bactéries connues dans une archive, permettant un accès rapide aux différentes souches de bactéries en fonction du cas particulier de mise en oeuvre dudit procédé de bioremédiation.

28. Mélange d'enzymes destiné à dégrader des hydrocarbures et permettant de mettre en oeuvre le procédé de bioremédiation qui permet une biodégradation accélérée d'hydrocarbures d'huile minérale dans des régions polaires couvertes par une banquise, en réalisant une bioaugmentation avec des bactéries exogènes capables de dégrader des hydrocarbures et en ajoutant des éléments nutritifs, selon l'une des revendications 6 à 13, 15 à 19 et 21 à 22,
**caractérisé par**
une association de différentes enzymes ou compositions d'enzymes pouvant être obtenues à partir desdites souches bactéries permettant d'obtenir les mélanges de bactéries selon l'une des revendications 23 à 27, lesdites souches de bactéries étant sélectionnées telles qu'elles maintiennent leur activité à une température ambiante de -3 °C, sont tolérantes vis-à-vis de différentes plages de température, sont tolérantes vis-à-vis de différentes plages de salinité, se distinguent les unes des autres en ce qui concerne leur capacité à dégrader des matières, et se distinguent les une des autres en ce qui concernent leur capacité à émulsifier l'huile.
